# EUROPÄISCHE PATENTSCHRIFT

(11) **EP 2 385 818 B1**
(45) Veröffentlichungstag und Bekanntmachung des Hinweises auf die Patenterteilung: **26.02.2014**
(21) Anmeldenummer: 09798887.7
(22) Anmeldetag: 18.12.2009
(51) Int. Cl.: A61K 8/34, A61Q 15/00

(54) **DIOLE ALS ANTITRANSPIRANTWIRKSAME MITTEL**
DIOLS AS ANTIPERSPIRANT AGENTS
DIOLS UTILISÉS COMME AGENTS ANTITRANSPIRANTS

(30) Priorität: 07.01.2009 DE 102009004269
(43) Veröffentlichungstag der Anmeldung: 16.11.2011
(73) Patentinhaber: Beiersdorf Aktiengesellschaft, 20253 Hamburg (DE)
(72) Erfinder: BIEL, Stefan, 21077 Hamburg (DE); MIERTSCH, Heike, 22459 Hamburg (DE); MÄTZOLD, Katja, 22607 Hamburg (DE); MEYER, Maren, 22459 Hamburg (DE); WEINERT, Katrin, 22763 Hamburg (DE)
(86) Internationale Anmeldenummer: PCT/EP2009/009105
(87) Internationale Veröffentlichungsnummer: WO 2010/078932

(56) Entgegenhaltungen:
- EP-A1- 1 604 642
- GB-A- 1 472 536
- US-A- 6 149 897

## Beschreibung

Die Erfindung betrifft Diole als antitranspirant wirksame Mittel und deren Verwendung in kosmetischen oder dermatologischen Zubereitungen.

Kosmetische Formulierungen zur Verwendung als Unterarmprodukte enthalten antitranspirante und/oder desodorierende Wirkstoffe.
Die desodorienden Wirkstoffe dienen als Schutz vor unangenehmen Gerüchen durch das Schwitzen. Verschiedenen Wirkprinzipen können den desodorierenden Wirkstoffen zu Grunde liegen. Denkbare Wirkprinzipen sind die Überdeckung des Schweißes z.B. mit Parfüm oder die Absorption des Geruches durch z.B. 1,5-Pentandiol (WO 2007/063065 A1). Da der Schweißgeruch durch die Zersetzung humaner Körperausscheidungen durch Bakterien entsteht, werden auch keimhemmende, antimikrobielle Wirkstoffe und Enzyminhibitoren als desodoriende Wirkstoffe bezeichnet und in kosmetischen Zubereitungen eingesetzt (Domsch, A., Die kosmetischen Präparate,1990, Band 11, 4. Auflage, Verlag für chem. Industrie, H. Ziolkowsky KG, Augsburg; Umbach, W., Kosmetik, 1995, 2. erweiterte Auflage, George Thieme Verlag, Stuttgart, New York).
Als Antitranspirantien werden Wirkstoffe bezeichnet, die das Austreten des Schweißes an die Oberfläche behindern. Dieses kann durch die Verschließung des Schweißdrüsenausführganges z.B. durch Filmbildner oder astringierende Wirkstoffe geschehen. Des Weiteren werde auch Substanzen, die die Aktivität der ekkrinen Schweißdrüse beeinflussen, als Antitranspirant-Wirkstoffe verwendet. (Domsch, A., Die kosmetischen Präparate,1990, Band II, 4. Auflage, Verlag für chem. Industrie, H. Ziolkowsky KG, Augsburg; Umbach, W., Kosmetik, 1995, 2. erweiterte Auflage, George Thieme Verlag, Stuttgart, New York).
Es ist damit klar zwischen desodorierend und antitranspirant wirkend zu unterscheiden.
Auch die Kombination von Adstringentien mit antimikrobiell wirksamen Stoffen in ein und derselben Zusammensetzung ist gebräuchlich.

Heutzutage beinhalten nahezu alle kosmetischen Antitranspirantien Astringentien - vorwiegend Aluminiumsalz, wie das Aluminiumhydroxychlorid (Aluminiumchlorhydrat, ACH oder aktiviertes ACH-AACH) oder Aluminium-Zirkonium-Salz (AZG), als antitranspiranten Wirkstoff.

Diese auf Aluminiumsalzen basierenden AT-Mittel reduzieren zwar die vom Körper abgegebene Schweißmenge, führen aber als ungewollter Nebeneffekt zu Hautreizungen sowie zu weißen Rückständen auf der Haut oder Kleidung nach dem Eintrocknen bzw. bei Langzeitanwendung zu Ablagerungen in der Kleidung. Außerdem führen der niedrige pH-Wert (< 5) und die Elektrolyteigenschaften der als Antitranspirant einsetzbaren Aluminiumsalze zu Einschränkungen bei der Herstellung kosmetischer Formulierungen für die Anwendung auf der Haut, sowohl hinsichtlich der Rohstoffauswahl (Säureverträglichkeit; keine starken Basen, da diese zur Ausfällung von Al(OH)₃ führen) als auch hinsichtlich der Lagerstabilität.

Wünschenswert wäre es demnach ein Antitranspirant zur Verfügung zu stellen, das die vorgenannten Nachteile und Nebenwirkungen nicht aufzeigt.

Wünschenswert wäre es weiterhin eine Antitranspirantzubereitung zur Verfügung zu stellen, die den Stand der Technik bereichert und eine Alternative zu den bekannten Zubereitungen darstellt.

Insbesondere ist es wünschenswert eine kosmetische und/oder dermatologische Antitranspirant-Formulierung mit einer Antitranspirantwirkung bereitzustellen ohne die Nachteile aluminiumhaltiger Zubereitungen aufzuweisen.
Insbesondere ist es wünschenswert eine solche Zubereitung zur Verfügung zu stellen, die zur Stabilisierung und/oder Aufrechterhaltung der Antitranspirantwirkung eines Antitranspirants nach dem Schwitzen und/oder Hautreinigung beiträgt.
Wünschenswert wäre zudem ein Antitranspirant, das im Gegensatz zu ACH-haltigen Systemen zusätzlich zur schweißhemmenden Wirkung eine Hautbefeuchtung der Achselhaut ermöglicht, da ACH-haltige Zubereitungen häufig ein Austrocknung und damit verbundenen zusätzlichen Reizung der Haut verursachen.

Insbesondere ist es auch wünschenswert einen Antitranspirantwirkstoff zur Verfügung zu stellen, der möglichst breite Möglichkeiten der galenischen Einarbeitung in kosmetisch akzeptable und attraktive Formelsysteme ermöglicht.

Bekannt ist, dass Glykole als Formelbestandteile in kosmetischen Zubereitungen und u.a. auch in Kombination mit ACH eingesetzt werden, wie beispielsweise in EP 1206 239 B1,

US 7235229 B2, US 2007/0202062 oder EP 1 206 237 B1 beschrieben.

In EP 1478 231 B1 und DE 199 24 496 A1 werden längerkettige Diole (C5 -C10) als antimikrobiell wirksame, also desodoriende Stoffe beschrieben und deren Einsatz als Deodorant und Konservierungsmittelhelfer. Des Weiteren werden Diole als Geruchsreduzierer und Absorbern in Sanitärartikeln und Sprays eingesetzt (WO 2007/063065).

AT 345467 und US 4617185 offenbaren organische Verbindungen, gewählt aus aliphatischen Verbindungen mit 2 bis 10 C-atomen bzw. 2 bis 3 C-atomen und wenigstens 2 Hydroxygruppen, mit den Geruch unterdrückenden Eigenschaften.
DE 3248093 offenbart die desodorierenden Eigenschaften von Propylenglykol.

Eine schweißhemmende, antitranspirant Wirkung der Diole wird im Stand der Technik nicht festgestellt. Vielmehr wird deren Kombination erst mit schweißhemmenden Wirkstoffen (Antitranspirantien) als bevorzugt offenbart.

Bekannt ist aus parallelen Anmeldungen der Anmelderin wie WO 2009/007089 A2 die Antitranspirantwirksamkeit kurzkettiger vicinaler Diole, Glykole, wie 1,2-Propylenglykol.

Aluminiumhydroxychlorid-Propylenglykol-Komplex sind ebenfalls bekannte AT-Mittel, wie u.a. in US 6251 412 B1 und US 6783027 B2 erwähnt.
Dieser Komplex ist ein Koordinationskomplex aus ACH und Propylenglykol bei dem Wasser, welches normalerweise am Metall koordiniert ist, durch Propylenglykol verdrängt und ersetzt wird. Der Anteil an ACH ist in diesen Komplexen deutlich größer als an Propylenglykol.
EP 1604642 A1 offenbart den Einsatz C3-C6-alkan Polyole mit mindestens 3 Hydroxygruppen in Antitranspirantien.

Aus all diesen Informationen des Standes der Technik war jedoch der Weg zur vorliegenden Erfindung nicht vorgezeigt.

Überraschend wurde nun herausgefunden, dass Diole der Struktur 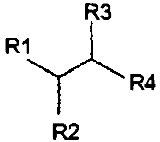 wobei die Reste R gewählt werden aus
R1 = 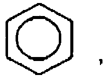 CH3, H,
R2, R3 und R4 = OH, CH2 - OH, CH3, CHOH - CH3, CH2-CHOH-CH3, C2H4-OH, C2H5, H,
wobei zwei OH-Gruppen im Molekül vorhanden, die maximale C- Anzahl aller Reste R2, R3 und R4 nicht größer als 6 ist und aliphatische vicinale Diole mit einer C-Atomanzahl von 2 bis 6 ausgenommen sind eine antitranspirant- und/oder schweißhemmenden Wirkung aufweisen.

Bevorzugte AT-Wirkstoffe sind 1,3-Propylenglykol, 1,3-Butylenglykol, 2,4-Pentylenglykol, 2,5-Hexandiol.

Beispielsweise bei 1,3 Propylenglykol sind R1 = H, R2 = OH, R3 = H und R4 = OH, bei 2,5 - Hexandiol sind R1 = CH3, R2 = OH, R3 = H, R4 = CH2-CHOH-CH3.

Auch aromatische Diole der Struktur 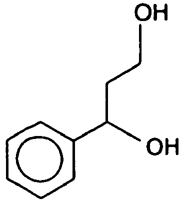 oder 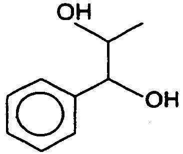 weisen eine schweißhemmende Wirkung auf.

Bei den obigen Strukturen sind
R1 = Phenyl,
   R2 = OH
   R3=H
   R4 = CH2 - OH bzw.
R1 = Phenyl,
   R2 = OH
   R3 = OH
   R4 = CH3.

Die erfindungsgemäßen Diole weisen, entsprechend dem Namen, mindestens und maximal nur zwei Hydroxygruppen auf. Eine Kettenlänge von mehr als 6 C - atomen bei aliphatischen Diolen ist ebenfalls nicht bevorzugt.
Handelt es sich um aromatische Diole, so sind die Reste R2, R3 und R4 in der Summe ebenfalls auf Ketten bis zu C6 begrenzt, vorteilhaft auf Kettenlängen von bis zu C4, insbesondere C3.

Erfindungsgemäß sind aliphatische vicinale Diole mit C2 bis C6, insbesondere Monoethylenglycol, Diethylenglycol, Propylenglykol (Propan-1,2-diol), Butylenglykol (1,2-Butylenglycol) sowie die entsprechenden 1,2 - Diole wie Pentandiol oder Hexandiol, ausgenommen.

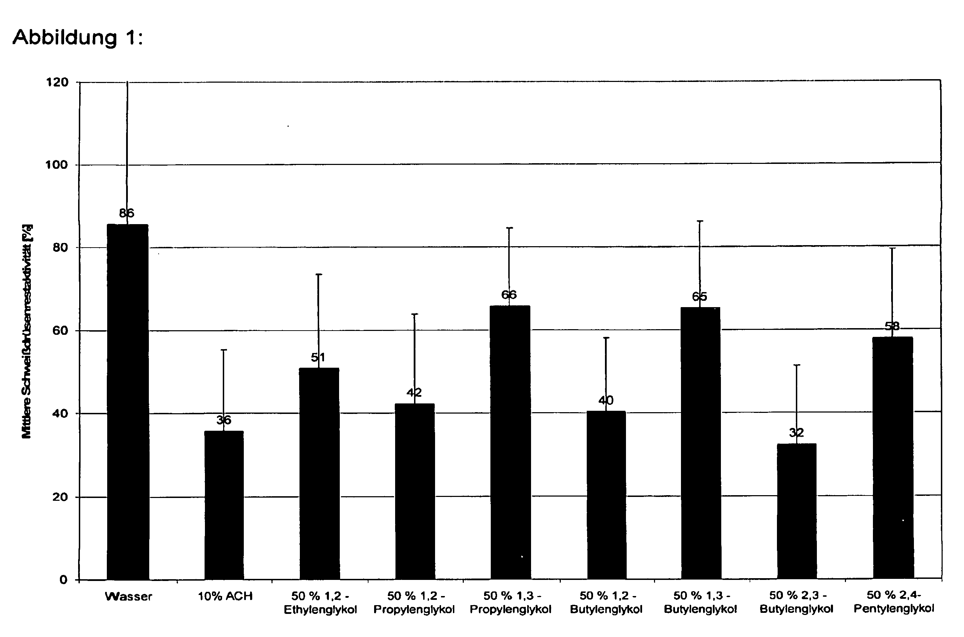

Die überraschende Antitranspirantwirksamkeit wurde in Studien zum AT-Wirkmechanismus der aromatischen Diole in wässrigen Systemen, unterschiedlichen Ölen und O/W-Emulsion untersucht (siehe Abbildung 1). In den Abbildungen ist die Restschweißmenge im Vergleich zum unbehandelten Areal nach Produktapplikation dargestellt. 100% Restschweißmenge entsprechen 0% Schweißreduktion. Die Schweißreduktion wurde in Anlehnung an M. Keskin et al. J. Invest. Dermatol. 1998; 110:591 gemessen. Bei den Messungen werden die Testsubstanzen auf dem Unterarm der Probanden angewendet. Die Probanden werden bei 75 °C zum Schwitzen angeregt; nach Beginn der Schweißproduktion wird mittels Silikonabdrucktechnik die Anzahl der schweißabgebenden Schweißdrüsen im Testareal ermittelt und gegen ein unbehandeltes Vergleichsareal abgeglichen.

Überraschenderweise zeigte sich in diesen Untersuchungen, dass auch die nicht vicinalen Diole der erfindungsgemäßen Struktur, wie insbesondere 1,3-Propylenglykol, 1,3-Butylenglykol, 2,4-Pentylenglykol und/oder 2,5 Hexandiol antitranspirant bzw. schweißhemmende Wirkung aufweisen.

Dies ist umso mehr erstaunlich, dass die AT-Leistung einer Zubereitung umfassend 1,3 Propylenglykol, vorteilhaft bis zu 50 % Gew. in Wasser dispergiert bezogen auf die Gesamtmasse der Zubereitung, eine gute AT-Leistung aufweist, im Vergleich zu einer Zubereitung umfassend 10 Gew.% Aluminiumchlorohydrat (siehe Abbildung 1).

In Abbildung 1 ist die Restschweißmenge im Vergleich zum unbehandelten Areal nach Produktapplikation im Vergleich zu den bekannten AT-wirkstoffen Aluminiumchlorohydrat sowie vicinalen Glykolen in Wasser, dargestellt. Die schweißhemmende Wirkung der erfindungsgemäßen Diole ist annähernd vergleichbar mit der vicinaler Diole, 1,2Propylenglykol und 2,3-Butylenglykol, wie in den parallelen Anmeldungen beschrieben.

D.h. die erfindungsgemäße Verwendung der erfindungsgemäßen Diole ermöglicht eine gleichwertige oder leicht schlechtere schweißhemmende Wirkung als bekannte und bewährte Zubereitung mit antitranspirantem Wirkstoff wie beispielsweise mit ACH.

In allen Abbildungen sieht man sehr deutlich, dass ohne bislang bekannte antitranspirant wirksame Stoffe, wie ACH, dennoch eine schweißhemmende Wirkung nur allein aufgrund der Diole zu beobachten ist.

Bevorzugt ist, die erfindunsgemäßen Diole in Kombination mit den antitranspirant wirksamen vicinalen Diolen zusammen in kosmetischen Zubereitungen einzusetzen.

Die Diole penetrieren sehr gut durch das Stratum Corneum und die Epidermis. Sie sind in der Lage einen osmotischen Druck aufzubauen, der den Mechanismus der Schweißsekretion beeinflusst. Die Resorption von Wasser entlang des Schweißdrüsenausführganges wird ebenfalls durch die erfindungsgemäßen Zubereitungen und die Diole beeinflusst.
Des Weiteren werden die Diole von dem Enzym Alkoholdehydrogenase in der Haut umgesetzt. Die dadurch entstehenden Reaktionsprodukte können die Membranstrukturen verändern und so eine veränderte Ionenpermeabilität verursachen, die sich wiederum auf die Schweißsekretion auswirken kann.

Vorteilhaft ist, dass in erfindungsgemäßen kosmetischen Zubereitungen umfassend die erfindungsgemäßen Diole, vornehmlich 1,3-Propylenglykol, 1,3-Butylenglykol, 2,4-Pentylenglykol und/oder 2,5 Hexandiol, gänzlich auf andere Antitranspirantwirkstoffe wie Aluminiumsalze, z.B. ACH, verzichten kann und dennoch ein ausreichender Antitranspirantschutz gewährleistet bleibt.

Dies führt auch zur Behebung der aufgeführten Nachteile, wie Hautreizung und Haut- bzw. Kleidungsverfärbungen (Weißeln), da diese vor allem auf die Gegenwart von ACH zurück zuführen sind.
Bevorzugt umfassen erfindungsgemäße kosmetische Zubereitung daher neben den Diolen keine weiteren astringierende oder antitranspirant wirksamen Stoffe, insbesondere keine Aluminiumsalze, insbesondere kein ACH und/oder AACH (aktiviertes Aluminiumchlorohydrat).

Bekannt ist, dass Zubereitungen mit Al-Salzen eine relativ dauerhafte Verstopfung der Schweißpore herbeiführen und daher auch eine AT-Wirksamkeit über mehrere Stunden bzw. Tage aufweisen können. D.h., dass auch am Tag nach der Applikation noch eine Wirkung vorhanden sein kann. Nachteilig ist aber, wie erläutert, dass es dabei zu Hautirritationen kommen kann.

Die Diole, insbesondere 1,3-Propylenglykol, 1,3-Butylenglykol, 2,4-Pentylenglykol und/oder 2,5 Hexandiol, zeigen nun erstaunlicherweise eine unmittelbare AT-Wirkung (Sofort-Effekt), die bereits nach dem ersten Auftragen messbar ist und nicht durch mehrfache Applikation aufgebaut werden muss. Dies jedoch ohne den Hautreizungen fördernden Depot-Effekt der Aluminiumsalze.
Wesentlicher Vorteil der erfindungsgemäßen Zubereitungen ist darüber hinaus, dass sich gegenüber den auf Aluminiumsalzen basierenden AT-Mitteln, keinerlei Verfärbungen auf der Haut oder Kleidung zeigt. Das sogenannte Weißeln unterbleibt ebenso wie die nach mehrfachem Tragen und Waschen in direkt auf der Achselhaut aufliegenden Textilien zu beobachtenden Rückstände.

Eine Kombination aus den Diolen mit schweißreduzierenden Mitteln, die nicht auf einem astringierenden Wirkmechanismus beruhen, ist vorteilhaft. Bevorzugt werden AT-mittel aus der Gruppen der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid (Glycopiperolat) gewählt, insbesondere können diese zu einem Anteil von bevorzugt 0,05 bis 1,0 Gew.%, vorzugsweise 0,1% - 0,7%, insbesondere 0,3% - 0,5 Gew.%, bezogen auf die Gesamtmasse der Zubereitung, zugesetzt werden. Eine Verstärkung der antitranspiranten Wirkung ist damit erreichbar.
Ebenfalls bevorzugt ist die Kombination mit antimikrobiellen Wirkstoffen, insbesondere mit Polyaminopropylenbiguanid und Kombinationen von bekannten desodorierenden und/oder antimikrobiellen Wirkstoffen

Als Antitranspirantwirkung wird die Möglichkeit der Verminderung oder Verhinderung der Schweißbildung verstanden. D.h. Erfindungsgemäße Diole, insbesondere 1,3-Propylenglykol, 1,3-Butylenglykol, 2,4-Pentylenglykol und/oder 2,5 Hexandiol wirken als Schweißhemmer, vermindern oder verhindern also die Schweißbildung.

Die erfindungsgemäßen Zubereitungen reduzieren den Schweißfluss (gemessen in Anlehung an G.E. Piérard et al., Skin Pharmacol Appl Skin Physiol 2003;16:324-342 und M. Keskin et al., J. Invest. Dermatol. 1998; 110:591) schon in geringen Konzentrationen in Zubereitungen analog den Beispielrezepturen.

Selbst nach starkem Schwitzen oder Körperreinigung ist noch eine etwas verminderte Schweißfluss-Reduktion zu beobachten, ohne dass vorher erneut Produkt aufgetragen wurde.
Die erfindungsgemäßen Diole sind zwar bekannte chemische Substanzen. In kosmetischen Zubereitungen und insbesondere als Antitranspirant wirksame Substanzen sind sie jedoch nicht benannt. Eine Verwendung in AT- bzw. Desodorantien als AT-Wirkstoff ist ebenfalls nicht bekannt.

Vorteilhaft können erfindungsgemäßen Zubereitungen zusätzlich auch Desodorantien zugesetzt werden.
Alle für Desodorantien gängigen Wirkstoffe können vorteilhaft zusätzlich genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Famesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden. Als deosodierende Wirkstoffe können in kosmetischen Zubereitungen auch geringe Mengen an Adstringenzien, z.B. Alaune eingesetzt werden. Die keimhemmende Wirkung von geringen Mengen an adstringierenden Substanzen wurde bereits im Patent EP 96 02108 beschrieben. Vorteilhaft sind dabei Konzentration zwischen 1 % und 8 %.
Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Bevorzugt ist die Kombination mit Detergenzien, wie z.B. Konservierungsmittel, Ethanol oder Parfümkompositionen, die zu einer Verstärkung der antitranspiranten Wirksamkeit der Diole führen.
Ebenso ist der Zusatz von Penetrationsfördernden Stoffen, wie Ethanol, zur erfindungsgemäßen Zubereitung bevorzugt. Die Einsatzkonzentration von Ethanol liegt dabei zwischen 0 und 50 Gew.%, bevorzugt zwischen 10 und 50 Gew.%, bezogen auf die Gesamtmasse der Zubereitung. Es konnte dabei ein verstärkender Einfluss des penetrationsfördernden Stoffes, wie Ethanol, auf die AT-Wirksamkeit der Diole festgestellt werden.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, W/Si, Si/W oder multiple Emulsion, Makro-, Mikro- oder Nanoemulsion), einer Dispersion, einer Pickering-Emulsion, eines Gels, eines Hydrodispersionsgels oder einer wasserfreien Zubereitung vorliegt.
Die Zubereitung kann erfindungsgemäß auch in Form einer dünnflüssigen, sprühfähigen wässrigen oder wässrig-alkoholischen Lösung, in Form eines Gels, in einer Wachsmatrix, einer Stiftform, als Salbe, Creme oder Lotion (ggf. sprühbar) vorliegen.
Die Zubereitung kann erfindungsgemäß vorteilhaft auch als Aerosol, Spray oder als Tränkungsmedium für ein Pflaster oder Tuch eingesetzt werden. Daher sind auch Pflaster und Tücher getränkt mit der erfindungsgemäßen Zubereitung, erfindungsgemäß.
Als kosmetische Zubereitung werden die erfindungsgemäßen Diole bevorzugt formuliert als Gel, Stift, Aerosol und/oder als Emulsion. Als Gel wird der Vorteil generiert, dass auch Gelbildner verwendet werden können, die ansonsten in Kombination mit ACH nicht verwendet werden können. Als Stift oder Aerosol bietet sich der Vorteil der Vermeidung von weißen Rückständen auf Haut und Kleidung. Als Emulsion formuliert bietet die erfindungsgemäße Zubereitung gegenüber ACH-haltigen Formulierungen ein besseres Hautgefühl und ermöglicht eine bessere Hautbefeuchtung.

Zur Applikation der Zubereitung lassen sich vorteilhaft herkömmliche Packmittel für kosmetische Zubereitungen, insbesondere Desodorantien und/oder Antitranspirantien verwenden, z. B. Aerosole, Zerstäuber, Stiftdispenser, Geldispenser, Tuben und Roller.

Die angegebenen Zahlenwerte in den Beispielen beziehen sich auf Gewichtsprozent in Bezug zur Gesamtmasse der Zubereitung.

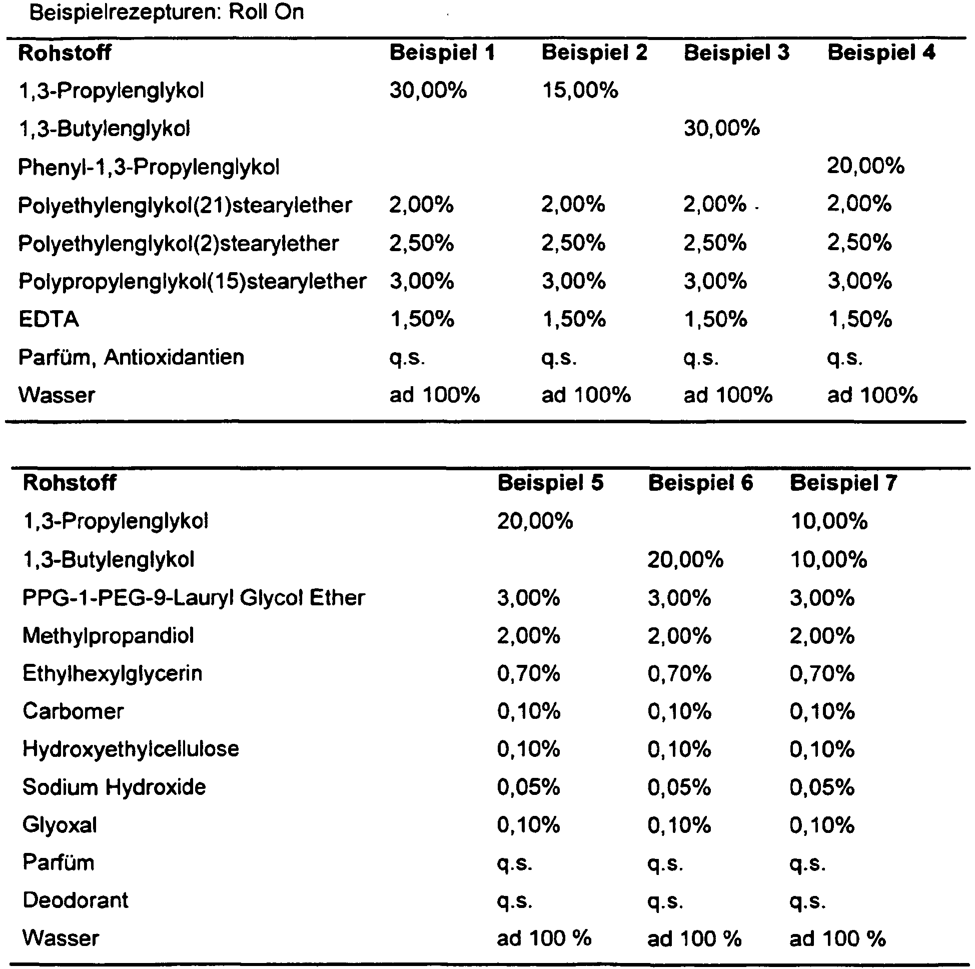

### Beispielrezeptur: Aerosole

| **Rohstoff** | **Beispiel 8** | **Beispiel 9** | **Beispiel 10** |
|---|---|---|---|
| 1,3-Propylenglykol | 30,00% | | 30,00% |
| 2,5 Hexandiol | | 30,00% | |
| Caprylic/Capric Triglyceride | 10,00% | 10,00% | 10,00% |
| Dicaprylyl Ether | 5,00% | 5,00% | 5,00% |
| PEG-45/Dodecyl Glycol Copolymer | 1,50% | 1,50% | |
| PEG-22/Dodecyl Glycol Copolymer | | | 1,20% |
| Polyglyceryl-3 Diisotearate | 1,50% | 1,50% | 1,20% |
| Magnesium Sulfate | | | 0,20% |
| Parfüm, Anitoxidantien | q.s. | | q.s. |
| Deodorant | q.s. | | q.s. |
| Wasser | ad 100% | ad 100% | ad 100% |

| | | | |
|---|---|---|---|
| Abfüllverhältnis: 30 wt.% Wirkstofflösung in 70 wt.% Propan/Butan/Isobutan | | | |

### Beispielrezeptur: Zerstäuber

| **Rohstoff** | **Beispiel 11** | **Beispiel12** | **Beispiel 13** |
|---|---|---|---|
| 1,3-Propylenglykol | 20,00% | | 10,00% |
| 2,5 Hexandiol | | 20,00% | 10,00% |
| Isoceteth-20 | 4,80% | | 4,80% |
| Dicaprylyl Ether | 3,00% | | 3,00% |
| Glycerly Isostearate | 2,40% | | 2,40% |
| Polyether-1 | 1,00% | | 1,00% |
| Deodorant | q.s. | | q.s. |
| Parfüm | q.s. | | q.s. |
| Wasser | ad 100 % | | ad 100 % |

## Patentansprüche

1. Verwendung von Diolen der Struktur 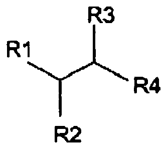 wobei die Reste R gewählt werden aus
R1 = 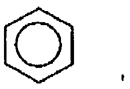 CH3, H,
R2, R3 und R4 = OH, CH2 - OH, CH3, CHOH - CH3, CH2-CHOH-CH3, C2H4-OH, C2H5, H,
wobei zwei OH-Gruppen im Molekül vorhanden sind, die maximale C- Anzahl aller Reste R2, R3 und R4 nicht größer als 6 ist und vicinale Diole mit einer C-Atomanzahl von 2 bis 6 ausgenommen sind, als antitranspirant- und/oder schweißhemmender Wirkstoff.

2. Verwendung nach Anspruch 1 **dadurch gekennzeichnet, dass** 1,3-Propylenglykol, 1,3-Butylenglykol, 2,4-Pentylenglykol und/oder 2,5 Hexandiol als antitranspirant- und/oder schweißhemmenden Wirkstoff gewählt wird.

3. Verwendung nach Anspruch 1 oder 2, **dadurch gekennzeichnet, dass** die Diole in topisch applizierbaren, insbesondere kosmetischen, Zubereitungen enthalten sind.

4. Verwendung nach Anspruch 3, **dadurch gekennzeichnet, dass** die Diole zu 10 bis 50 Gew.%, insbesondere 15 bis 30 Gew.%, bezogen auf die Gesamtmasse der Zubereitung, enthalten sind.

5. Verwendung nach einem der Ansprüche 3 oder 4, **dadurch gekennzeichnet, dass** die Diole in Kombination mit anderen antitranspirant wirksamen Diolen eingesetzt werden.

6. Verwendung nach einem der Ansprüche 1 bis 2 als alleiniger Antitranspirantwirkstoff in kosmetischen Zubereitungen.

## Claims

1. Use of diols of the structure 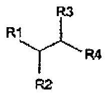 where the radicals R are selected from
R1 = 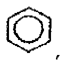 CH3, H,
R2, R3 and R4 = OH, CH2-OH, CH3, CHOH-CH3, CH2-CHOH-CH3, C2H4-OH, C2H5, H,
where two OH groups are present in the molecule, the maximum carbon number of all radicals R2, R3 and R4 is not greater than 6 and vicinal diols with a carbon atom number of 2 to 6 are excluded, as antiperspirant and/or perspiration-inhibiting active ingredient.

2. Use according to Claim 1, **characterized in that** 1,3-propylene glycol, 1,3-butylene glycol, 2,4-pentylene glycol and/or 2,5-hexanediol is selected as antiperspirant and/or perspiration-inhibiting active ingredient.

3. Use according to Claim 1 or 2, **characterized in that** the diols are present in topically applicable, in particular cosmetic, preparations.

4. Use according to Claim 3, **characterized in that** the diols are present to 10 to 50% by weight, in particular 15 to 30% by weight, based on the total mass of the preparation.

5. Use according to one of Claims 3 and 4, **characterized in that** the diols are used in combination with other antiperspirant diols.

6. Use according to one of Claims 1 to 2 as the sole antiperspirant active ingredient in cosmetic preparations.

## Revendications

1. Utilisation de diols de structure 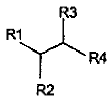 dans laquelle les radicaux R sont choisis parmi
R1 = 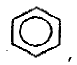 CH₃, H,
R2, R3 et R4 = OH, CH₂-OH, CH₃, CHOH-CH₃, CH₂-CHOH-CH₃, C₂H₄-OH, C₂H₅, H,
deux groupes OH étant présents dans la molécule, le nombre maximum d'atomes de carbone de tous les radicaux R2, R3 et R4 n'étant pas supérieur à 6 et les diols vicinaux ayant un nombre d'atomes de carbone de 2 à 6 étant exclus, en tant des substance active anti-transpirante et/ou antisudorale.

2. Utilisation selon la revendication 1, **caractérisée en ce qu'**on choisit comme substance active anti-transpirante et/ou antisudorale le 1,3-propylène-glycol, le 1,3-butylèneglycol, le 2,4-pentylèneglycol et/ou le 2,5-hexanediol.

3. Utilisation selon la revendication 1 ou 2, **caractérisée en ce que** les diols sont contenus dans des préparations, en particulier cosmétiques, applicables localement.

4. Utilisation selon la revendication 3, **caractérisée en ce que** les diols sont contenus à raison de 10 à 50 % en poids, en particulier de 15 à 30 % en poids, par rapport à la masse totale de la préparation.

5. Utilisation selon l'une quelconque des revendications 3 et 4, **caractérisée en ce qu'**on utilise les diols en association avec d'autres diols à activité anti-transpirante.

6. Utilisation selon l'une quelconque des revendications 1 et 2, en tant qu'unique substance active anti-transpirante dans des préparations cosmétiques.
